# EUROPEAN PATENT SPECIFICATION

(11) **EP 1 127 881 B1**
(45) Date of publication and mention of the grant of the patent: **08.08.2012**
(21) Application number: 01104114.2
(22) Date of filing: 21.02.2001
(51) Int. Cl.: C07D 249/20, C08K 5/3475

(54) **Alkylation process and novel hydroxyphenylbenztriazoles**
Alkylierungsverfahren und Hydroxyphenylbenztriazole
Procédé d'alkylation et hydroxy-phényl-benzotriazoles

(30) Priority: 21.02.2000 EP 00103592
(43) Date of publication of application: 29.08.2001
(73) Proprietor: DSM IP Assets B.V., 6411 TE Heerlen (NL)
(72) Inventor: Huber, Ulrich, 8703 Erlenbach (CH)
(74) Representative: Berg, Katja

(56) References cited:
- GB-A- 2 077 747
- US-A- 4 587 346
- US-A- 4 812 575
- B.D. TIFFANY: "Partial para-migration in the allylic rearrangement of o-acetamidophenyl allyl ether and of o-aminophenyl allyl ether" JOURNAL OF THE AMERICAN CHEMICAL SOCIETY., vol. 70, 1948, pages 592-594, XP002166678 DC US

## Description

The invention relates to a process for alkylating certain 2-hydroxyphenyl-benztriazoles which are effective in absorbing ultra violet radiation. In a further aspect, the invention relates to novel alkylated 2-hydroxyphenyl-benztriazoles, to novel cosmetic or dermatological sunscreen compositions containing alkylated 2-hydroxyphenyl-benztriazoles, and to their use as UV screening agents.

US patent No. 4 587 346 discloses a process for alkylating 2-(2-hydroxy-5-methylphenyl)-2H-benzotriazoles by reaction with alkene compounds in the presence of a catalyst. The process produces random mixtures of isomers.

UV screening compositions comprising 2-hydroxyphenyl-benztriazoles are described in the European patent publications EP 0 711 778 A. and EP 0 392 883 A, US patents Nos. 4 316 033 and 4 349 602, and International patent application WO 94/06404.

It has been found that alkylated 2-(2-hydroxy-5-methylphenyl)-2H-benzotriazoles can be obtained in substantially pure form. Further, it has been found that certain novel 2-hydroxyphenyl-benztriazoles have improved solubilty and extinction and are more economical to prepare than those of the prior art quoted above.

The compounds of the general formula I 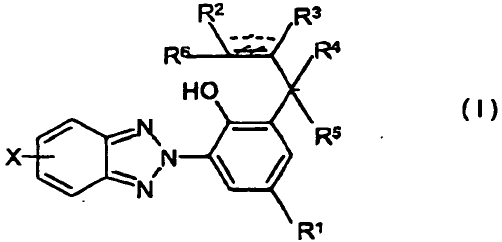 wherein
R¹ is alkyl; R², R³, R⁴ R⁵ and R⁶ are, independently, hydrogen, alkyl or alkenyl; X is hydrogen, halogen, alkyl or alkoxy; and the dotted bond is an optional bond,
   with the proviso that one of R4 and R5 is alkyl having at least 2 carbon atoms or is alkenyl,
   may be prepared by a process, which comprises the steps of
   a) reacting a compound of the general formula III 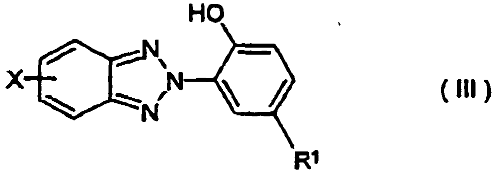 with a compound of the general formula IV 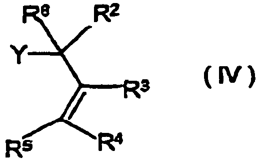 to obtain a compound of the general formula II 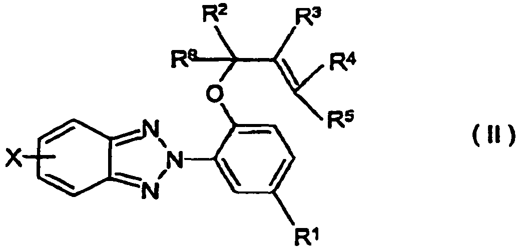 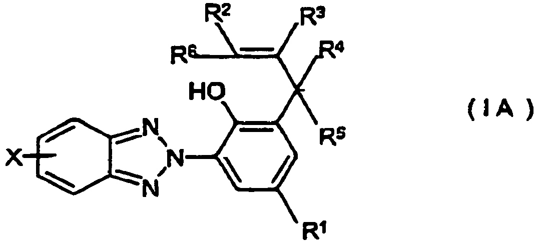
   b) heating the compound of the general formula II obtained in step a) to yield a compound of the general formula I A, and, if desired,
   c) hydrogenating the double bond in the compound of the general formula I A to obtain a compound of the general formula I wherein the dotted bond is absent;
wherein in the above formulae IA, II, III and IV, R¹, R², R³, R⁴ R⁵, R⁶, X and the dotted bond are as defined in formula I, and Y is a leaving group.

Of the compounds of the general formula I above, the following compounds
2-(Benzotriazole-2-yl)-4-methyl-6-(1-octen-3-yl)-phenol,
2-(Benzotriazole-2-yl)-4-(1,1,3,3-tetramethylbutyl)-6-(1-hexen-3-yl)-phenol,
2-(Benzotriazole-2-yl)-4-methyl-6-(1-decen-3-yl)-phenol,
2-(Benzotriazole-2-yl)-4-methyl-6-(1-hexen-3-yl)-phenol,
2-(Benzotrianole-2-yl)-4-methyl-6-(1-dodecen-3-yl)-phenol,
2-(Benzotriazole-2-yl)-4-methyl-6-(1-hexadecen-3-yl)phenol,
2-(Benzotriazole-2 yl)-4-(1,1,3,3-tetramethylbutyl)-6-(3-hexyl)-phenol,
2-(Benzotriazole-2-yl)-4-methyl-6-(3-hexyl)-phenol,
2-(Benzotriazole-2-yl)-4-methyl-6-(3,7-dimtethyl-3-octyl)-phenol,
2-(Benzotriazole-2-yl)-4-methyl-6-(3,7-dimethyl-1-octen-3-yl)-phenol, and
are novel compounds and as such are also an object of the present invention.

A further aspect of the present invention is a process for the manufacture of these novel compounds according to claim 10.

In still another aspect, the invention relates to a novel cosmetic or dermatological light screening composition comprising a compound of the general formula I 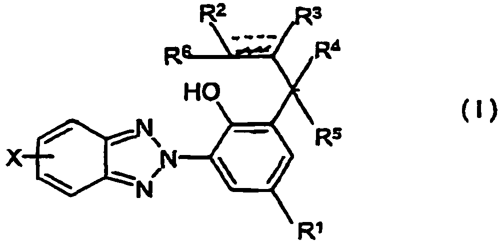 wherein R¹ is alkyl; R², R³, R⁴ R⁵ and R⁶ are, independently, hydrogen, alkyl or alkenyl; X is hydrogen, halogen, alkyl or alkoxy; and the dotted bond is an optional bond,
with the proviso that one of R⁴ and R⁵ is alkyl having at least 2 carbon atoms or is alkenyl;
and a carrier material conventionally used in such compositions. Preferred compounds of the general formula I in these cosmetic or dermatological light screening compositions are those according to claims 2 to 3. In yet another aspect, the invention relates to novel UV light screening compositions containing a 2-hydroxyphenyl-benztriazole of the general formula I above, wherein X, R¹, R², R³, R⁴ R⁵ and R⁶ and the dotted bond are as defined in claim 1, with the proviso that one of R⁴ and R⁷ is alkyl having at least 2 carbon atoms or is alkenyl; and at least one additional UV-A and/or UV-B screening agent. Finally, the present invention relates to the use of the novel 2-hydroxyphenyl-benztriazoles as mentioned above as UV light screening agents.

Especially preferred compounds for use as UV light screening agents are
2-(Benzotriazole-2-yl)-4-methyl-6-(1-octen-3-yl)-phenol,
2-(Benzotriazole-2-yl)-4-(1,1,3,3-tetramethylbutyl)-6-(1-hexen-3-yl)-phenol, and
2-(Benzotriazole-2-yl)-4-(1,1,3,3-tetramethylbutyl)-6-(3-hexyl)-phenol.
Most preferred is 2-(Benzotriazole-2-yl)-4-methyl-6-(1-octen-3-yl)-phenol.

As used herein the term alkyl denotes saturated straight or branched chain hydrocarbon groups containing 1 to 21, preferably 1 to 8 carbon atoms, such as methyl, ethyl, propyl, isopropyl, butyl, sec. butyl, isobutyl, pentyl neopentyl, hexyl, 2-ethyl-hexyl, and octyl. Similarly, the term alkoxy denotes saturated straight or branched chain hydrocarbon groups which are bound through an oxygen atom and which contain 1 to 21, preferably 1 to 8 carbon atoms, such as methoxy, ethoxy, propoxy, isopropoxy, butoxy, sec. butoxy, isobutoxy, pentyloxy, neopentyloxy, hexyloxy, 2-ethyl-hexyloxy and octyloxy. The term alkenyl denotes straight or branched chain hydrocarbon groups containing at least one double bond and 2 to 21, preferably 2 to 8 carbon atoms. Examples of such alkenyl groups are propen-2-yl, propen-3-yl, buten-3-yl, buten-4-yl, penten-4-yl, and penten-5-yl. The term halogen denotes fluoro, chloro, bromo and iodo.

A preferred group of compounds within formula I are those wherein the dotted bond is present. Also preferred are compounds of formula I wherein R⁵ and R⁶ are hydrogen. Further preferred are compounds wherein one of R² to R⁴ is alkyl having at least three carbon atoms and the other two of R² to R⁴ are hydrogen. The total number of carbon atoms in R² to R⁶ is preferably 3 to 21, more particularly 3 to 9 carbon atoms, most preferably 3 to 5. Especially preferred are compounds of formula I wherein R² and R³ are hydrogen and R⁴ is alkyl having 3 to 5 carbon atoms. R¹ is preferably methyl or 1,1,3,3-tetramethylbutyl. X is preferably hydrogen, methoxy or chloro, and most preferably hydrogen.

The compounds of the general formula I may be prepared as shown in Scheme 1 below:

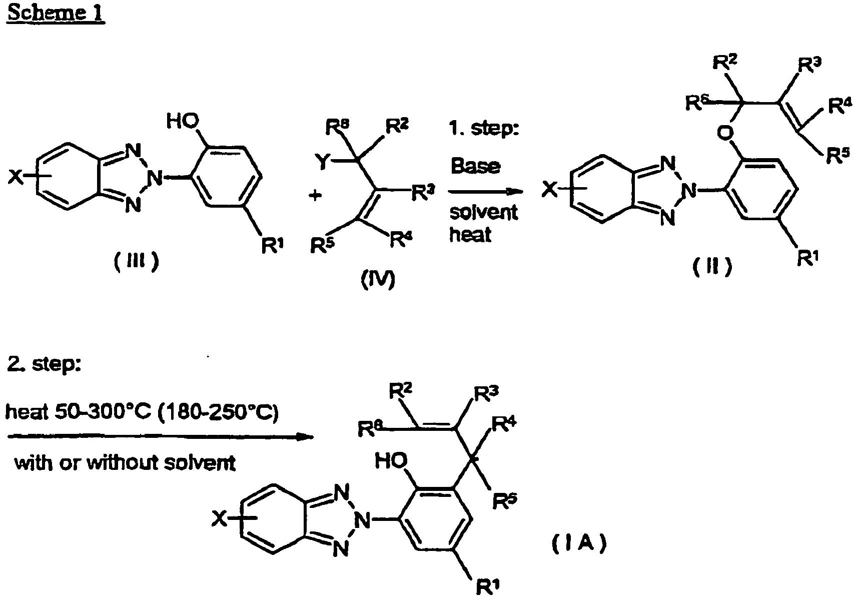

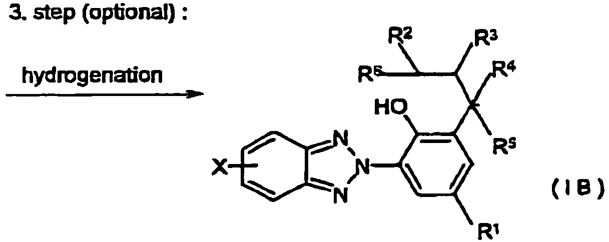

In the first reaction step the benzotriazolyl phenol is reacted with an alkene compound carrying a leaving group Y, such as halogen, e.g., chloro, bromo, or a sulfonyloxy group, e.g., tosyloxy or mesyloxy. The reaction can be carried out in a manner known per se for the alkenylation of phenolic hydroxy groups, i.e., in the presence of a base such as an alkali carbonate, e.g., sodium carbonate, alkali hydroxide or alkali alcoholates, e.g., sodium methylate; an amine such as triethyl amine, N,N-dimethylamino pyridine or 1,4-diazabicyclo[2.2.2]octane (DABCO); in a polar solvent e.g., an alcohol such as n-butanol, an ether such as diethyleneglycol monomethyl ether, tetrahydrofuran or dioxan; or in dimethyl formamide, dimethyl sulfoxide, N,N-dimethyl propylene urea or 1-methyl pyrrolidone, or in a solvent which simultaneously may serve as a base, such as N,N-dimethylamino pyridine, at temperatures from room temperature up to the boiling point of the reaction mixture. The phenol ether obtained can be rearranged by heating, suitably to a temperature of from 50 to 300 °C, if desired, in a solvent, suitably in a solvent that is conventionally used in Claisen rearrangements, e.g., in diethyl aniline or trichloro benzene to afford the corresponding compound of formula I wherein the dotted bond is present. The olefinic double bond can be hydrogenated in a manner known per se, e.g., with elemental hydrogen in the presence of a noble metal catalyst such as Pd, or with Rany-Ni, preferably with elemental hydrogen in the presence of an appropriate catalyst which does not attack the triazole ring, e.g., a partially inactivated noble metal catalyst such as a Lindlar catalyst.

The starting compounds of formulae III and IV are known or can be prepared by methods known per se or described hereinafter. For instance, compounds of formula III can be prepared by a reaction sequence that comprises converting an X-substituted o-nitro aniline by reaction with sodium nitrite to the corresponding diazonium salt followed by a diazotation reaction with a R1-substituted phenol to form the diazo compound and reduction of the remaining nitro group with concomitant cyclisation to form the triazole ring. Compounds of formula IV can be prepared following Scheme 2 :

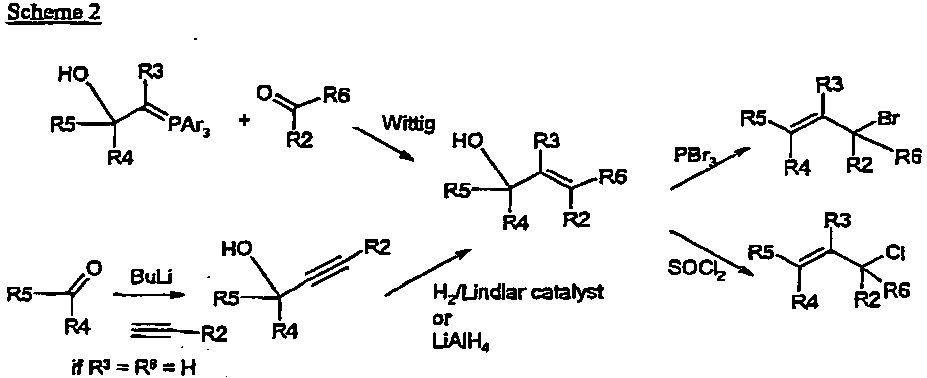

The phenol ether derivatives of formula II obtained in the first reaction step of the production of the novel compounds of claim 9 are also novel compounds and as such are also an object of the present invention.

The novel compounds of the general formula I have adsorption maxima in both the UV-A and the UV-B region. They have a good liposolubility and photostability.

For the preparation of light screening agents, especially of preparations for dermatological or cosmetic use, such as skin protection and sunscreen formulations for everyday cosmetics a compound of formula I can be incorporated in auxiliary agents, e.g. a cosmetic base, which are conventionally used for such formulations. Where convenient, other conventional UV-A and/or UV-B screening agents may also be added. The preparation of said light screening agents is well known to the skilled artisan in this field. The amount of compounds of the general formula I and other known UV-filters is not critical. Suitable amounts are about 0.5 to about 12% of active ingredient, i.e., a compound of the general formula I and, if desired, any additional UV-A or UV-B screening agent.

Examples of UV B screening agents, i.e. substances having absorption maxima between about 290 and 320 nm, which come into consideration for combination with the compounds of the present invention are for example the following organic and inorganic compounds :
- Acrylates such as 2-ethylhexyl 2-cyano-3,3-diphenylacrylate (octocrylene, PARSOL® 340), ethyl 2-cyano-3,3-diphenylacrylate and the like;
--- Camphor derivatives such as 4-methyl benzylidene camphor (PARSOL® 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like;
--- Cinnamate derivatives such as octyl methoxycinnamate (PARSOL® MCX), ethoxyethyl methoxycinnamate, diethanolamine methoxycinnamate (PARSOL® Hydro), isoamyl methoxycinnamate and the like as well as cinnamic acid derivatives bond to siloxanes;
--- Organosiloxane compounds containing benzmalonate groups as described in the European Patent Publications EP 0358584 B1, EP 0538431 B1 and EP 0709080 A1;
--- Pigments such as microparticulated TiO₂, and the like. The term "microparticulated" refers to a particle size from about 5 nm to about 200 nm, particularly from about 15 nm to about 100 nm. The TiO₂ particles may also be coated by metal oxides such as e.g. aluminum or zirconium oxides or by organic coatings such as e.g. polyols, methicone, aluminum stearate, alkyl silane. Such coatings are well known in the art.
--- Imidazole derivatives such as e.g. 2-phenyl benzimidazole sulfonic acid and its salts (PARSOL® HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like.
--- Salicylate derivatives such as isopropylbenzyl salicylate, benzyl salicylate, butyl salicylate, octyl salicylate (NEO HELIOPAN OS), isooctyl salicylate or homomenthyl salicylate (homosalate, HELIOPAN) and the like;
--- Triazone derivatives such as octyl triazone (UVINUL T-150), dioctyl butamido triazone (UVASORB HEB) and the like.

Examples of UV A screening agents i.e. substances having absorption maxima between about 320 and 400 nm, which come into consideration for combination with the compounds of the present invention are for example the following organic and inorganic compounds:
---- Dibenzoylmethane derivatives such as 4-tert. butyl-4'-methoxydibenzoylmethane (PARSOL® 1789), dimethoxydibenzoylmethane, isopropyldibenzoylmethane and the like;
---- Benzotriazole derivatives such as 2,2'-methylene-bis-(6-(2H-benzotriazole-2-yl)-4-(1,1,3,3,-tetramethylbutyl)-phenol (TINOSORB M) and the like;
---- Pigments such as microparticulated ZnO and the like. The term "microparticulated" refers to a particle size from about 5 nm to about 200 nm, particularly from about 15 nm to about 100 nm. The ZnO particles may also be coated by metal oxides such as e.g. aluminum or zirconium oxides or by organic coatings such as e.g. polyols, methicone, aluminum stearate, alkyl silane. Such coatings are well known in the art.

As dibenzoylmethane derivatives are photolabile it may be desirable to photostabilize these UV-A screening agents. Thus, the term "conventional UV-A screening agent" also refers to dibenzoylmethane derivatives such as e.g. PARSOL® 1789 stabilized by, e.g.,
--- 3,3-Diphenylacrylate derivatives as described in the European Patent Publications EP 0 514 491 B1 and EP 0 780 119 A1;
---- Benzylidene camphor derivatives as described in the US Patent No. 5,605,680;
---- Organosiloxanes containing benzmalonate groups as described in the European Patent Publications EP 0358584 B1, EP 0538431 B1 and EP 0709080 A1.

As cosmetic bases conventional for light screening compositions in the scope of the present invention there can be used any conventional preparation which corresponds to the cosmetic requirements, e.g. creams, lotions, emulsions, salves, gels, solutions, sprays, sticks and milks; see also: Sunscreens, Development, Evaluation and Regulatory Aspects, ed. N.Y. Lowe, N.A. Shaath, Marcel Dekker, Inc. New York and Basel, 1990. Having regard to their good lipophility, the compounds of the general formula I can be incorporated well into oil and fat containing cosmetic preparations.

The following Examples illustrate the invention in more detail. In the Examples, tlc. means thin layer chromatography.

### Example 1

a) To a mixture of 7g of 2-(benzotriazole-2-yl)-4-methyl-6-phenol (Tinuvin® P, CIBA SA.) in 35 ml of 1-methyl-pyrrolidone, 8.2g of anhydrous sodium carbonate and 2 mg of potassium iodide. 8.5g (44.7 mmol) of 1-bromo-2-octene (prepared from 1-octene-3-ol by the method of L. Miginiac and B. Mauzé, Bull. Soc. Chim. France 1968, 2544, 2547) was added slowly under nitrogen atmosphere. The reaction mixture was left to stir for two hours at room temperature and for 18 hours at 100°C. The reaction was traced by tlc. (hexane : ethylacetate = 3:1). The reaction mixture was then cooled to 20°C, poored on water and extracted (3x) with ethyl acetate. The combined organic phases were washed with water, 2n NaOH (2x) and brine and dried over sodium sulfate. After concentration and drying in high vacuum 10.7g of crude liquid 2-(2-oct-2-enyloxy-5-methyl-phenyl)-2H-benzotriazole as identified by NMR and MS was obtained.
b) 4g (11.9 mmol) of 2-(2-oct-2-enyloxy-5-methyl-phenyl)-2H-benzotriazole prepared as described above in 5 ml of N,N-diethylaniline were heated to reflux (230°C.)under nitrogen atmosphere. The reaction was traced by tlc. (hexane : ethyl acetate = 2:1). After 165 min the reaction was complete, and a solution of 2n HCl was added to the cold reaction mixture, followed by extraction with ether (3x). The combined organic phases were washed with a 10% KOH solution, and the latter acidified to pH 3-4 with 5n HCl and extracted with ether. The combined ether phases were dried over sodium sulfate and concentrated to yield 3.75g (94%) of a brown liquid, which contained 8% of 2-(benzotriazole-2-yl)-4-methyl-6-(3-octen-2-yl)-phenol. These 8% of the byproduct were identified by NMR (CDCl₃): 0.87 ppm (Tr/3Pr); 1.26-1.4 (M/7Pr); 2.05 (D x Tr/2Pr); 2.38 (S/3Pr); 4.03 (Q x D/1Pr); 5.57 (D x Tr/1Pr); 5.72 (D x D x Tr/1Pr); 7.05 (D/1Pr); 7.46 (M/2Pr); 7.90 (M/2Pr); 8.07 (D/1Pr) and 11.4 (S/1Pr), UV(CH₂Cl₂) 306 and 344 nm. After chromatography on silica gel (Merck) with hexane/ diethyl ether slightly yellow crystals of the main product were formed. M.p. 37-38°C. UV(CH₂Cl₂) 306 nm (16'800) and 344 nm (16'555); MS: 335 (M⁺), 264, 145 (100%), 117; NMR (CDCl₃): 0.87 ppm (Tr/3Pr); 1.26-1.4 (M/6Pr); 1.77 (D x Tr/2Pr); 2.38 (S/3Pr); 3.90 (D x Tr/1Pr); 5.05 (D/1Pr); 5.10 (D/1Pr); 6.04 (D x D x D/1Pr); 7.05 (D/1Pr); 7.46 (M/2Pr); 7.90 (M/2Pr); 8.07 (D/1Pr) and 11.4 (S/1Pr).

In a separate reaction 1g of the above starting material was heated without solvent in a Kugelrohr oven for 270 min to 220°C. Practically pure 2-Benzotriazole-2-yl-4-methyl-6-(1-octen-3-yl)-phenol was obtained in quantitative yield.

The product is easily mixable with cosmetic solverts like e.g. Cétiol LC (Cocoyl caprylate caprate). When irradiated in high dilution with a Heraeus 150 W Hg-lamp the product has been shown to be photostable.

### Example 2

a) To 7.98g (24.7mmol) of 2-(benzotriazole-2-yl)-4-(1,1,3,3-tetramethyl-butyl)-6-phenol (Aldrich) in 40 ml of 1-methyl-pyrrolidone and 6.5g of anhydrous sodium carbonate, 5.8g (35.7 mmol) of 1-bromo-2-hexene (prepared from 1-hexene-3-ol by the method of L. Miginiac and B. Mauzé, Bull. Soc. Chim. France 1968, 2544, 2547) were added slowly under nitrogen atmosphere. The reaction mixture was left to stir for two hours at room temperature and for 18 hours at 80°C. The reaction was traced by tlc. (hexane : ether : CH₂Cl₂ = 3:1:1). The reaction mixture was then cooled to 20°C and distributed (3x) between water and ethyl acetate. The combined organic phases were washed with water, 2n NaOH (2x) and brine and dried over sodium sulfate. After concentration a crude crystalline product was obtained, which was recrystallised from hexane to yield 6.17g of 2-(2-hex-2-enyloxy-5-(1,1,3,3-tetramethylbutyl)-phenyl)-2H-benzotriazole, M.p. 80-81°C; UV(CH₂Cl₂): 287 nm (16'305); MS: 405 (M⁺), 323, 252 (100%).
b) 5.6g (13.8 mmol) of2-(2-hex-2-enyloxy-5-(1,1,3,3-tetramethylbutyl)-phenyl)-2H-benzotriazole prepared as described above in 100 ml of N,N-dimethylaniline were heated to reflux. The reaction was traced by tlc. (hexane : ethyl acetate = 7:3). After 22 hours the reaction was complete. N,N-dimethylaniline was destilled off and the product freed from residual amine in high vacuum to yield 4.7g (84%) of 2-(benzotriazole-2-yl)-4-(1,1,3,3-tetramethylbutyl)-6-(1-hexen-3-yl)-phenol as an orange liquid. UV(CH₂Cl₂): 305 nm (16'007) and 343 nm (14'359); MS: 405 (M⁺), 334 (100%).

The product is easily mixable with cosmetic solverts like e.g. Cétiol LC (Cocoyl caprylate caprate). When irradiated in high dilution with a Heraeus 150 W Hg-lamp the product has been shown to be photostable.

### Example 3

0.5g of 2-(benzotriazole-2-yl)-4-methyl-6-(1-octen-3-yl)-phenol and a trace of "Lindlar catalyst" (Fluka) in 20 ml of hexane was hydrogenated for 4 hours under normal pressure of hydrogen. Then the reaction mixture was filtered and concentrated to yield (quantitatively) 2-(benzotriazole-2-yl)-4-methyl-6-(3-octyl)-phenol as a yellow liquid. UV(CH₂Cl₂): 306 nm (14'602) and 346 nm (13'850); MS: 337 (M⁺), 308, 266, 238 (100%).

The product is easily mixable with cosmetic solverts like e.g. Cétiol LC (Cocoyl caprylate caprate). When irradiated in high dilution with a Heraeus 150 W Hg-lamp the product has been shown to be photostable.

### Example 4

2g of 2-(benzotriazole-2-yl)-4-(1,1,3,3-tetramethylbutyl)-6-(1-hexen-3-yl)-phenol and a trace of "Lindlar catalyst" (Fluka) in 25 ml of hexane was hydrogenated for 5 hours under normal pressure of hydrogen. Then the reaction mixture was filtered and concentrated to yield 2g of 2-(benzotriazole-2-yl)-4-(1,1,3,3-tetramethylbutyl)-6-(3-hexyl)-phenol as an orange liquid. UV(CH₂Cl₂): 306 nm (15'121) and 346 nm (13'120); MS: 407 (M⁺), 336 (100%).

The product is easily mixable with cosmetic solverts like e.g. Cétiol LC (Cocoyl caprylate caprate). When irradiated in high dilution with a Heraeus 150 W Hg-lamp the product has been shown to be photostable.

### Example 5

### Preparation of a O/W anionic sunscreen lotion UV-B and UV-A:

Broad spectrum sunscreen lotion containing 4% of a compound of example 4.

| **wt.%** | **compound** | **supplier** | **chemical name** |
|---|---|---|---|
| **Part A** | | | |
| 3 | PARSOL^{®} MCX | ¹⁾ | Octyl methoxycinnamate |
| 4 | product of example 4 | | |
| 3 | PARSOL^{®} 500 | ¹⁾ | 4-Methylbenzylidene camphor |
| 4 | PARSOL^{®} 1789 | ¹⁾ | 4-t-Butyl-4'-methoxy-dibenzoylmethane |
| 2 | Glyceryl monostearate | | Glyceryl stearate |
| 2 | Cetyl alcohol extra | | Cetyl alcohol |
| 2 | Ganex V-220 | ²⁾ | PVP/Eicosene copolymer |
| 4 | Ceraphyl 375 | ²⁾ | Isostearyl neopentanoate |
| 4 | Ceraphyl 847 | ²⁾ | Octyldodecyl stearoyl stearate |
| 2 | Amphisol K | ¹⁾ | Potassium cetylphosphate |
| 0.1 | Edeta BD | | Disodium EDTA |
| 0.6 | Phenonip | ³⁾ | Phenoxyethanol & Methyl-, Ethyl-, Propyl- & Butyl-paraben |

| **Part B** | | | |
|---|---|---|---|
| 11.15 | water deion. | | water deion. |
| 50 | Carbopol 9341% solution | ⁴⁾ | Carbomer |
| 5 | Propyleneglycol | | 1,2-Propanediol |
| 0.15 | Nipagin M | ³⁾ | Methylparaben |
| 3 | KOH(10%) | | Potassium hydroxyde |
| q.s. | Perfume oil | | Fragrance |

| | | | |
|---|---|---|---|
| Part A is heated in a reactor to 85°C. When homogeneous, add Part B, followed by addition of preheated KOH (75°C), cooling and degassing of the emulsion. | | | |

### Example 6

### Preparation of a O/W sunscreen lotion UV-B and UV-A:

Broad spectrum sunscreen lotion containing 2% of a compound of example 3.

| **wt.%** | **compound** | **supplier** | **chemical name** |
|---|---|---|---|
| 2 | PARSOL^{®} MCX | ¹⁾ | Octyl methoxycinnamate |
| 2 | product of example 3 | | |
| 3 | PARSOL^{®} 1789 | ¹⁾ | 4-t-Butyl-4'-methoxy-dibenzoylmethane |
| 12 | Cétiol LC | ⁶⁾ | Cocoyl-caprylate/caprate |
| 4 | Dermol 185 | ⁶⁾ | Isostearyl neopentanoate |
| 0.25 | Diethyleneglycol monostearate | | PEG-2-stearate |
| 1 | Cetylalcohol | | Cetylalcohol |
| 0.25 | MPOB/PPOB | | Methyl-propylparabene |
| 0.1 | EDTA BD | | EDTA-sodium salt |
| 1 | Amphisol DEA | ¹⁾ | Diethanolamine cetylphosphate |

| **Part B** | | | |
|---|---|---|---|
| 20 | Permulene TR-1 (+%) | ⁴⁾ | Acrylate C10-C30 Alkylacrylate |
| 48.6 | water deion. | | water deion. |
| 5 | Propyleneglycol | | 1,2-Propanediol |
| 0.8 | KOH(10%) | | Potassium hydroxyde |

| | | | |
|---|---|---|---|
| Part A is heated in a reactor to 85°C. Part B is slowly added within 10 min., followed by addition of KOH, cooling and degassing of the emulsion. | | | |

### Suppliers

1) F. HOFFMANN - LA ROCHE LTD, CH-4070 Basel / Switzerland
2) International Specialty Products ISP
3) NIPA LABORATORIES LTD, Mid Glam. - CF38 2SN / England
4) B.F. GOODRICH COMPANY, Brecksville - OH 44141 / USA
5) HENKEL K.G, Düsseldorf/Germany
6) BERNEL Chemical Co. INC. Englwood NJ. USA

## Claims

1. A cosmetic or dermatological light screening composition comprising a compound of the general formula I 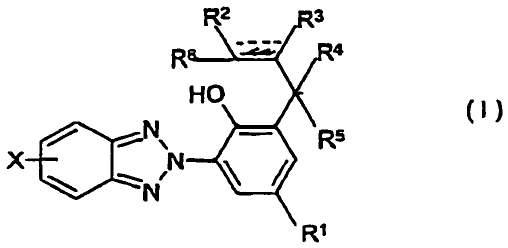
whereinR¹ is alkyl; R², R³, R⁴ R⁵ and R⁶ are, independently, hydrogen, alkyl or alkenyl; X is hydrogen, halogen, alkyl or alkoxy; and the dotted bond is an optional bond,
with the proviso that one of R⁴ and R⁵ is alkyl having at least 2 carbon atoms or is alkenyl; and a carrier material conventionally used in such compositions.

2. The composition as in claim 1 wherein the compound of formula I is selected from the group consisting of 2-(benzotriazole-2-yl)-4-methyl-6-(1-octen-3-yl)-phenol, 2-(benzotriazole-2-yl)-4-(1,1,3,3-tetramethylbutyl)-6-(1-hexen-3-yl)-phenol, 2-(benzotriazole-2-yl)-4-methyl-6-(1-decen-3-yl)-phenol, 2-(benzotriazole-2-yl)-4-methyl-6-(1-hexen-3-yl)-phenol, 2-(benzotriazole-2-yl)-4-methyl-6-(1-dodecen-3-yl)-phenol, 2-(benzotriazole-2-yl)-4-methyl-6-(1-hexadecen-3-yl)-phenol, 2-(benzotriazole-2-yl)-4-methyl-6-(3-octyl)-phenol, 2-(benzotriazole-2-yl)-4-(1,1,3,3-tetramethylbutyl)-6-(3-hexyl) phenol, 2-(benzotriazole-2-yl)-methyl-6-(3-hexyl)-phenol, 2-benzotriazole-2-yl)-4-methyl-6-(3-decyl)-phenol, 2-(benzotriazole-2-yl)-4-methyl-6(3-dodecyl)-phenol, 2-(benzotriazole-2-yl)-4-methyl-6-(3-hexadecyl)-phenol, 2-(benzotriazole-2-yl)-4-methyl-6-(3,7-dimethyl-3-octyl)-phenol and 2-(benzotriazole-2-yl)-4-methyl-6-(3,7-dimethyl-1-octen-3-yl)-phenol.

3. The composition as in claim 1 wherein the compound of the formula I is 2-(benzotriazole-2-yl)-4-methyl-6-(1-octen-3-yl)-phenol, 2-(benzotriazole-2-yl)-4-(1,1,3,3-tetramethylbutyl)-6-(1-hexen-3-yl)-phenol, 2-(benzotriazole-2-yl)-4-methyl-6-(3-octyl)-phenol or 2-(benzotriazole-2-yl)-4-(1,1,3,3-tetramethylbutyl)-6-(3-hexyl)-phenol.

4. The composition as any one of claims 1 to 3 which comprises at least one additional UV-A and/or UV-B screening agent.

5. Use of a compound selected from the group consisting of 2-(benzotriazole-2-yl)-4-methyl-6-(1-octen-3-yl)-phenol, 2-(benzotriazole-2-yl)-4-(1,1,3,3-tetramethylbutyl)-6-(1-hexen-3-yl)-phenol, 2-(benzotriazole-2-yl)-4-methyl-6-(1-decen-3-yl)-phenol, 2-(benzotriazole-2-yl)-4-methyl-6-(1-hexen-3-yl)-phenol, 2-(benzotriazole-2-yl)-4-methyl-6-(1-dodecen-3-yl)-phenol, 2-(benzotriazole-2-yl)-4-methyl-6-(1-hexadecen-3-yl)-phenol, 2-(benzotriazole-2-yl)-4-(1,1,3,3-tetramethylbutyl)-6-(3-hexyl)-phenol, 2-(benzotriazole-2-yl)-4-methyl-6-(3-hexyl)-phenol, 2-(benzotriazole-2-yl)-4-methyl-6-(3,7-dimethyl-3-octyl)-phenol and 2-(benzotriazole-2-yl)-4-methyl-6-(3,7-dimethyl-1-octen-3-yl)-phenol as a UV light screening agent.

6. The use as in claim 5 wherein the compound of the formula I is selected from the group consisting of 2-(benzotriazole-2-yl)-4-methyl-6-(1-octen-3-yl)-phenol, 2-(benzotriazole-2-yl)-4-(1,1,3,3-tetramethylbutyl)-6-(1-hexen-3-yl)-phenol, and 2-(benzotriazole-2-yl)-4-(1,1,3,3-tetramethylbutyl)-6-(3-hexyl)-phenol.

7. Compound selected from the group consisting of 2-(benzotriazole-2-yl)-4-methyl-6-(1-octen-3-yl)-phenol, 2-(benzotriazole-2-yl)-4-(1,1,3,3-tetramethybutyl)-6-(1-hexen-3-yl)-phenol, 2-(benzotriazole-2-yl)-4-methyl-6-(1-decen-3-yl)-phenol, 2-(benzotriazole-2-yl)-4-methyl-6-(1-hexen-3-yl)-phenol, 2-(benzotriazole-2-yl)-4-methyl-6-(1-dodecen-3-yl)-phenol, 2-(benzotriazole-2-yl)-4-methyl-6-(1-hexadecen-3-yl)-phenol, 2-(benzotriazole-2-yl)-4-(1,1,3,3-tetramethylbutyl)-6-(3-hexyl)-phenol, 2-(benzotriazole-2-yl)-4-methyl-6-(3-hexyl)-phenol, 2-(benzotriazole-2-yl)-4-methyl-6-(3,7-dimethyl-3-octyl)-phenol and 2-(benzotriazole-2-yl)-4-methyl-6-(3,7-dimethyl-1-octen-3-yl)-phenol.

8. Process for the preparation of the compounds of claim 7 which comprises the steps of
a) reacting a compound of the general formula III. 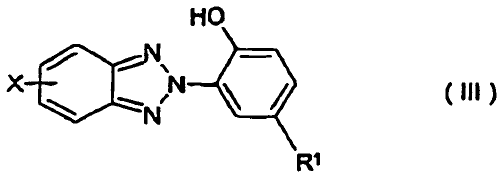
wherein X is hydrogen and R¹ is methyl or 1,1,3,3-tetramethylbutyl, with a compound of the general formula IV, 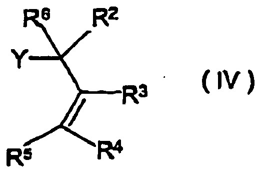
wherein R² is hydrogen, R³ is hydrogen, one of R⁴ and R⁵ is ethyl or vinyl and the other is hydrogen or methyl, R⁶ is methyl, n-propyl, 2-methylpropyl, n-pentyl, n-heptyl, n-undecyl, to obtain a compound of the general formula II 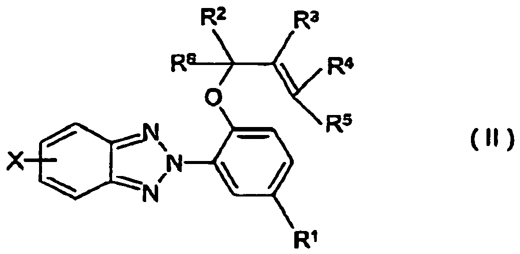
b) heating the compound of the general formula **II** obtained in step a) to yield a compound of the general formula I A 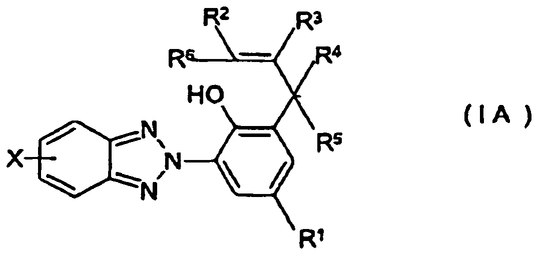 and, c) hydrogenating the double bond in the compound of the general formula I A;
wherein in the above formulae IA and II R¹, R², R³, R⁴, R⁵, R⁶ and X are as defined above for formulae III and IV, and Y is a leaving group.

9. Compounds of the formula II as defined in claim 8.

## Patentansprüche

1. Kosmetische oder dermatologische Lichtschutzzusammensetzung, umfassend
eine Verbindung der allgemeinen Formel I 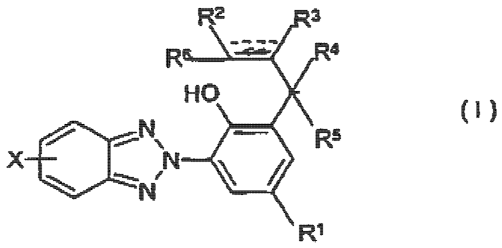
worin R¹ für Alkyl steht; R^{2,} R³, R⁴, R⁵ und R⁶ unabhängig voneinander für Wasserstoff, Alkyl oder Alkenyl stehen; X für Wasserstoff, Halogen, Alkyl oder Alkoxy steht und die gestrichelte Bindung eine fakultative Bindung darstellt,
mit der Maßgabe, dass einer der Reste R⁴ und R⁵ für Alkyl mit mindestens 2 Kohlenstoffatomen oder für Alkenyl steht;
und ein in derartigen Zusammensetzungen herkömmlicherweise verwendetes Trägermaterial.

2. Zusammensetzung nach Anspruch 1, wobei die Verbindung der Formel (I) aus der Gruppe bestehend aus 2-(Benzotriazol-2-yl)-4-methyl-6-(1-octen-3-yl)phenol, 2-(Benzotriazol-2-yl)-4-(1,1,3,3-tetramethylbutyl)-6-(1-hexen-3-yl)phenol, 2-(Benzotriazol-2-yl)-4-methyl-6-(1-decen-3-yl)phenol, 2-(Benzotriazol-2-yl)-4-methyl-6-(1-hexen-3-yl)-phenol, 2-(Benzotriazol-2-yl)-4-methyl-6-(1-dodecen-3-yl)phenol, 2-(Benzotriazol-2-yl)-4-methyl-6-(1-hexadecen-3-yl)phenol, 2-(Benzotriazol-2-yl)-4-methyl-6-(3-octyl)phenol, 2-(Benzotriazol-2-yl)-4-(1,1,3,3-tetramethylbutyl)-6-(3-hexyl)phenol, 2-(Benzotriazol-2-yl)-4-methyl-6-(3-hexyl)phenol, 2-(Benzotriazol-2-yl)-4-methyl-6-(3-decyl)phenol, 2-(Benzotriazol-2-yl)-4-methyl-6-(3-dodecyl)phenol, 2-(Benzotriazol-2-yl)-4-methyl-6-(3-hexadecyl)phenol, 2-(Benzotriazol-2-yl)-4-methyl-6-(3,7-dimethyl-3-octyl)phenol und 2-(Benzotriazol-2-yl)-4-methyl-6-(3,7-dimethyl-1-octen-3yl)phenol ausgewählt ist.

3. Zusammensetzung nach Anspruch 1, wobei es sich bei der Verbindung der Formel (I) um 2-(Benzotriazol-2-yl)-4-methyl-6-(1-octen-3-yl)phenol, 2-(Benzotriazol-2-yl)-4-(1,1,3,3-tetramethylbutyl)-6-(1-hexen-3-yl)phenol, 2-(Benzotriazol-2-yl)-4-methyl-6-(3-octyl)phenol oder 2-(Benzotriazol-2-yl)-4-(1,1,3,3-tetramethylbutyl)-6-(3-hexyl)phenol handelt.

4. Zusammensetzung nach einem der Ansprüche 1 bis 3, die mindestens ein zusätzliches UV-A- und/oder UV-B-Lichtschutzmittel umfasst.

5. Verwendung einer Verbindung aus der Gruppe bestehend aus 2-(Benzotriazol-2-yl)-4-methyl-6-(1-octen-3-yl)phenol, 2-(Benzotriazol-2-yl)-4-(1,1,3,3-tetramethylbutyl)-6-(1-hexen-3-yl)phenol, 2-(Benzotriazol-2-yl)-4-methyl-6-(1-decen-3-yl)-phenol, 2-(Benzotriazol-2-yl)-4-methyl-6-(1-hexen-3-yl)phenol, 2-(Benzotriazol-2-yl)-4-methyl-6-(1-dodecen-3-yl)phenol, 2-(Benzotriazol-2-yl)-4-methyl-6-(1-hexadecen-yl)phenol, 2-(Benzotriazol-2-yl)-4-(1,1,3,3-tetramethylbutyl)-6-(3-hexyl)phenol, 2-(Benzotriazol-2-yl)-4-methyl-6-(3-hexyl)phenol, 2-(Benzotriazol-2-yl)-4-methyl-6-(3,7-dimethyl-3-octyl)phenol und 2-(Benzotriazol-2-yl)-4-methyl-6-(3,7-dimethyl-1-octen-3yl)phenol als UV-Lichtschutzmittel.

6. Verwendung nach Anspruch 5, wobei die Verbindung der Formel (I) aus der Gruppe bestehend aus 2-(Benzotriazol-2-yl)-4-methyl-6-(1-octen-3-yl)-phenol, 2-(Benzotriazol-2-yl)-4-(1,1,3,3-tetramethylbutyl)-6-(1-hexen-3-yl)phenol und 2-(Benzotriazol-2-yl)-4-(1,1,3,3-tetramethylbutyl)-6-(3-hexyl)phenol ausgewählt ist.

7. Verbindung aus der Gruppe bestehend aus 2-(Benzotriazol-2-yl)-4-methyl-6-(1-octen-3-yl)phenol, 2-(Benzotriazol-2-yl)-4-(1,1,3,3-tetramethylbutyl)-6-(1-hexen-3-yl)phenol, 2-(Benzotriazol-2-yl)-4-methyl-6-(1-decen-3-yl)phenol, 2-(Benzotriazol-2-yl)-4-methyl-6-(1-hexen-3-yl)-phenol, 2-(Benzotriazol-2-yl)-4-methyl-6-(1-dodecen-3-yl)phenol, 2-(Benzotriazol-2-yl)-4-methyl-6-(1-hexadecen-3-yl)phenol, 2-(Benzotriazol-2-yl)-4-(1,1,3,3-tetramethylbutyl)-6-(3-hexyl)phenol, 2-(Benzotriazol-2-yl)-4-methyl-6-(3-hexyl)phenol, 2-(Benzotriazol-2-yl)-4-methyl-6-(3,7-dimethyl-3-octyl)phenol, und 2-(Benzotriazol-2-yl)-4-methyl-6-(3,7-dimethyl-1-octen-3yl)phenol.

8. Verfahren zur Herstellung der Verbindungen gemäß Anspruch 7, bei dem man
a) eine Verbindung der allgemeinen Formel III, 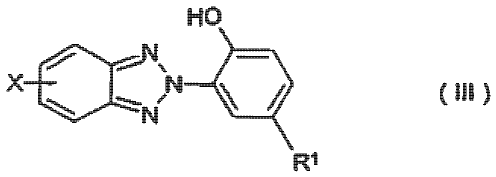
worin X für Wasserstoff steht und R¹ für Methyl oder 1,1,3,3-Tetramethylbutyl steht, mit einer Verbindung der allgemeinen Formel IV, 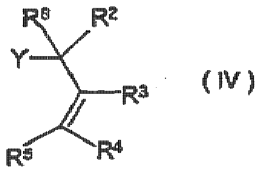
worin R² für Wasserstoff steht, R³ für Wasserstoff steht, einer der Reste R⁴ und R⁵ für Ethyl oder Vinyl steht und der andere für Wasserstoff oder Methyl steht, R⁶ für Methyl, n-Propyl, 2-Methylpropyl, n-Pentyl, n-Heptyl oder n-Undecyl steht, zu einer Verbindung der allgemeinen Formel (II) 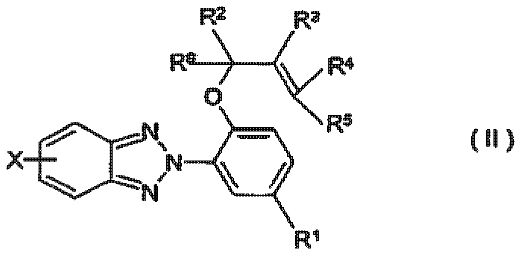
umsetzt;
b) die in Schritt a) erhaltene Verbindung der allgemeinen Formel II erhitzt, wobei man eine Verbindung der allgemeinen Formel IA 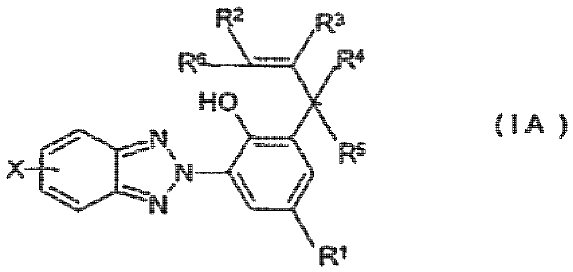 erhält; und
c) die Doppelbindung in der Verbindung der allgemeinen Formel IA hydriert;
wobei in den obigen Formeln IA und II R¹, R², R³, R⁴, R⁵, R⁶ und X die oben für die Formeln III und IV angegebene Bedeutung besitzen und Y für eine Abgangsgruppe steht.

9. Verbindungen der Formel II gemäß Anspruch 8.

## Revendications

1. Composition cosmétique ou dermatologique antisolaire comprenant un composé de formule générale I 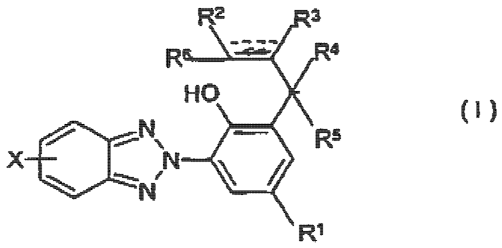 dans laquelle R¹ est alkyle ; R², R³, R⁴, R⁵ et R⁶ sont indépendamment hydrogène, alkyle ou alcényle ; X est hydrogène, halogène, alkyle ou alcoxy ; et la ligne en pointillés est une liaison facultative, à condition que l'un de R⁴ et R⁵ est alkyle ayant au moins 2 atomes de carbone ou est alcényle ; et une matière support utilisée de manière classique dans de telles compositions.

2. Composition selon la revendication 1, **caractérisée en ce que** le composé de formule I est choisi dans le groupe constitué par le 2-(benzotriazol-2-yl)-4-méthyl-6-(1-octèn-3-yl)-phénol, le 2-(benzotriazol-2-yl)-4-(1,1,3,3-tétraméthylbutyl)-6-(1-hexèn-3-yl)-phénol, le 2-benzotriazol-2-yl)-4-méthyl-6-(1-décèn-3-yl)-phénol, 2-(benzotriazol-2-yl)-4-méthyl-6-(1-hexèn-3-yl)-phénol, le 2-(benzotriazol-2-yl)-4-méthyl-6-(1-dodécèn-3-yl)-phénol, le 2-(benzotriazol-2-yl)-4-méthyl-6-(1-hexadécèn-3-yl)-phénol, le 2-(benzotriazol-2-yl)-4-méthyl-6-(3-octyl)-phénol, le 2-(benzotriazol-2-yl)-4-(1,1,3,3-tétraméthylbutyl)-6-(3-hexyl)-phénol, le 2-(benzotriazol-2-yl)-4-méthyl-6-(3-hexyl)-phénol, le 2-(benzotriazol-2-yl)-4-méthyl-6-(3-décyl)-phénol, le 2-(benzotriazol-2-yl)-4-méthyl-6-(3-dodécyl)-phénol, le 2-(benzotriazol-2-yl)-4-méthyl-6-(3-hexadécyl)-phénol, le 2-(benzotriazol-2-yl)-4-méthyl-6-(3,7-diméthyl-3-octyl)-phénol et le 2-(benzotriazol-2-yl)-4-méthyl-6-(3,7-diméthyl-1-octèn-3-yl)-phénol.

3. Composition selon la revendication 1, **caractérisée en ce que** le composé de formule I est le 2-(benzotriazol-2-yl)-4-méthyl-6-(1-octèn-3-yl)-phénol, le 2-(benzotriazol-2-yl)-4-(1,1,3,3-tétraméthylbutyl)-6-(1-hexèn-3-yl)-phénol, le 2-(benzotriazole-2-yl)-4-méthyl-6-(3-octyl)-phénol ou le le 2-(benzotriazol-2-yl)-4-(1,1,3,3-tétraméthylbutyl)-6-(3-hexyl)-phénol.

4. Composition selon l'une quelconque des revendications 1 à 3, **caractérisée en ce qu'**elle comprend au moins un autre filtre UV-A et/ou UV-B.

5. Utilisation d'un composé choisi dans le groupe constitué par le 2-(benzotriazol-2-yl)-4-méthyl-6-(1-octèn-3-yl)-phénol, le 2-(benzotriazol-2-yl)-4-(1,1,3,3-tétraméthylbutyl)-6-(1-hexèn-3-yl)-phénol, le 2-(benzotriazol-2-yl)-4-méthyl-6-(1-décèn-3-yl)-phénol, le 2-(benzotriazol-2-yl)-4-méthyl-6-(1-hexèn-3-yl)-phénol, le 2-(benzotriazol-2-yl)-4-méthyl-6-(1-dodécèn-3-yl)-phénol, le 2-(benzotriazol-2-yl)-4-méthyl-6-(1-hexadécèn-3-yl)-phénol, le 2-(benzotriazol-2-yl)-4-(1,1,3,3-tétraméthylbutyl)-6-(3-hexyl)-phénol, le 2-(benzotriazol-2-yl)-4-méthyl-6-(3-hexyl)-phénol, le 2-(benzotriazol-2-yl)-4-méthyl-6-(3-décyl)-phénol, le 2-(benzotriazol-2-yl)-4-méthyl-6-(3,7-diméthyl-3-octyl)-phénol et le 2-(benzotriazol-2-yl)-4-méthyl-6-(3,7-diméthyl-1-octèn-3-yl)-phénol en tant qu'agent de filtrage de la lumière ultraviolette.

6. Utilisation selon la revendication 5, **caractérisée en ce que** le composé de formule I est choisi dans le groupe constitué par le 2-(benzotriazol-2-yl)-4-méthyl-6-(1-octèn-3-yl)-phénol, le 2-(benzotriazol-2-yl)-4-(1,1,3,3-tétraméthylbutyl)-6-(1-hexèn-3-yl)-phénol et le 2-(benzotriazol-2-yl)-4-(1,1,3,3-tétraméthylbutyl)-6-(3-hexyl)-phénol.

7. Composé choisi dans le groupe constitué par le 2-(benzotriazol-2-yl)-4-méthyl-6-(1-octèn-3-yl)-phénol, le 2-(benzotriazol-2-yl)-4-(1,1,3,3-tétraméthylbutyl)-6-(1-hexèn-3-yl)-phénol, le 2-benzotriazol-2-yl)-4-méthyl-6-(1-décèn-3-yl)-phénol, le 2-(benzotriazol-2-yl)-4-méthyl-6-(1-hexèn-3-yl)-phénol, le 2-(benzotriazol-2-yl)-4-méthyl-6-(1-dodécèn-3-yl)-phénol, le 2-(benzotriazol-2-yl)-4-méthyl-6-(1-hexadécèn-3-yl)-phénol, le 2-(benzotriazol-2-yl)-4-(1,1,3,3-tétraméthylbutyl)-6-(3-hexyl)-phénol, le 2-(benzotriazol-2-yl)-4-méthyl-6-(3-hexyl)-phénol, le 2-(benzotriazol-2-yl)-4-méthyl-6-(3-décyl)-phénol, le 2-(benzotriazol-2-yl)-4-méthyl-6-(3,7-diméthyl-3-octyl)-phénol et le 2-(benzotriazol-2-yl)-4-méthyl-6-(3,7-diméthyl-1-octèn-3-yl)-phénol.

8. Procédé de préparation des composés selon la revendication 7, **caractérisé en ce qu'**il comprend les étapes consistant à
a) faire réagir un composé de formule générale III 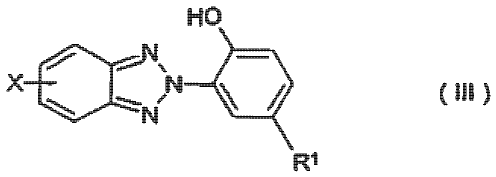
dans laquelle X est hydrogène et R¹ est méthyle ou 1,1,3,3-tétraméthylbutyle avec un composé de formule générale IV, 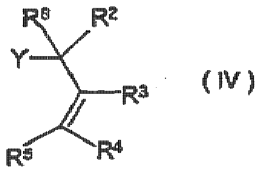
dans laquelle R² est hydrogène, R³ est hydrogène, l'un de R⁴ et R⁵ est éthyle ou vinyle et l'autre est hydrogène ou méthyle, R⁶ est méthyle, n-propyle, 2-méthylpropyle, n-pentyle, n-heptyle, n-undécyle, pour obtenir un composé de formule générale II 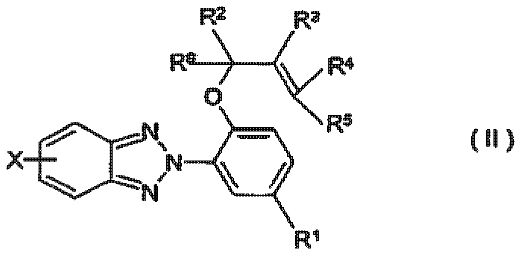
b) chauffer le composé de formule générale II obtenu à l'étape a) pour donner un composé de formule générale IA 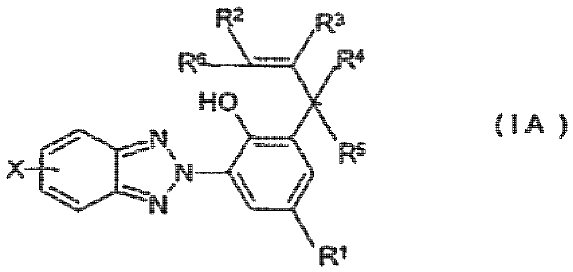 et,
c) hydrogéner la double liaison dans le composé de formule générale IA ; où dans les formules IA et II ci-dessus, R¹, R², R³, R⁴, R⁵, R⁶ et X sont tels que définis ci-dessus pour les formules III et IV, et Y est un groupement partant.

9. Composés de formule II tels que définis dans la revendication 8.
